# EUROPEAN PATENT APPLICATION

(11) **EP 0 648 745 A1**
(43) Date of publication of application: **19.04.1995**
(21) Application number: 94119942.4
(22) Date of filing: 01.08.1989
(51) Int. Cl.: C07D 213/38, A61K 31/44

(54) **Arylalkylamine having anticonvulsant and neuroprotective properties**

(30) Priority: 12.08.1988 US 232566
(62) Divisional of application: 89307815.4
(71) Applicant: Astra Aktiebolag, 151 85 Södertälje (SE)
(72) Inventor: Griffith, Ronald Conrad, Pittsford, New York 14534 (US); Napier, James Joseph, Lindenhurst, Illinois 60046 (US)
(74) Representative: England, Christopher David

(57) **Abstract**

The invention provides (*S*)-1-phenyl-2-(2-pyridinyl)ethylamine, and its pharmaceutically acceptable salts. The compound is indicated as an anticonvulsant and neuroprotective agent.

## Description

This invention relates to pharmaceutical compounds, some of which are novel, and to their anticonvulsant, sedative and neuroprotective properties.

Compounds which possess anticonvulsant, antihypoxic or N-methyl-(d)-aspartate (NMDA) blocking properties are useful in the treatment and/or prevention of neuro-degeneration in pathological conditions such as stroke, cerebral ischaemia, cerebral palsy, hypoglycaemia, epilepsy, Alzheimer's disease, Huntington's chorea, Olivo-pontocerebellar atrophy, perinatal asphyxia and anoxia. It has now been discovered that many substituted arylalkyl-amines and -amides possess anticonvulsant and neuroprotective properties which are useful for the treatment of such disorders. Many of these compounds also possess sedative properties.

Niemers *et al* [Synthesis, 9, 593-595 (1976)] disclose 1-phenyl-2-(2-pyridinyl)ethylamine. However, neither separation of its enantiomers nor particular salts are mentioned.

According to the invention we provide the use of a compound of formula I, 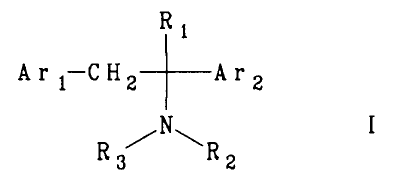
wherein,
Ar₁ and Ar₂, which may be the same or different, independently represent phenyl substituted by one or more of amino, nitro, chlorine, bromine, hydroxy, C1 to 6 alkoxy, C1 to 6 alkyl or cyano; in addition one of Ar₁ or Ar₂ may also represent phenyl;
R₁ represents hydrogen or C1 to 6 alkyl;
R₂ represents hydrogen or COCH₂NH₂;
R₃ represents hydrogen or C1 to 6 alkyl;
provided that when R₂ represents hydrogen, then one or both of Ar₁ and Ar₂ may also represent phenyl, fluorophenyl or 2-, 3- or 4- pyridinyl and R₁ may also represent C1 to 6 alkoxycarbonyl or trifluoromethyl;
or a pharmaceutically acceptable salt thereof.

This invention also relates to all stereoisomeric forms, optical enantiomeric forms and pharmaceutically acceptable acid addition salts of the compounds of formula I.

The compounds of formula I possess useful anticonvulsant properties as demonstrated by their ability to inhibit maximal electroshock (MES) induced seizures in mice. The compounds of formula I also possess antihypoxia activity as demonstrated by their ability to increase the survival time of mice in an oxygen depleted environment. In addition, compounds of formula I inhibit the onset of convulsions and death induced by administration of N-methyl-(d)-aspartate (NMDA) to mice.

### DETAILED DESCRIPTION

Some of the compounds of formula I are known compounds and are commercially available. Compounds of formula I in which R₂ represents hydrogen may be prepared by known procedures for amine formation, for example those given in "Advanced Organic Chemistry", 2^{nd} Ed., J March, (McGraw-Hill) at page 1172. Compounds of formula I in which R₂ represents COCH₂NH₂ may be prepared by known procedures for amide formation, for example those given in "Advanced Organic Chemistry" at page 1171.

Methods for the preparation of compounds of formula I are also given in US patent no.s 4,769,466 and 4,798,687.

A process for the preparation of compounds of formula I which may be specifically mentioned, and which is a further aspect of the invention, comprises:
a) for compounds of formula I in which R₂ represents hydrogen and R₃ represents C1 to 6 alkyl, alkylation of the corresponding compound of formula I in which R₃ represents hydrogen;
b) for compounds of formula I in which R₂ and R₃ both represent hydrogen, amide hydrolysis of the corresponding compound of formula II in which Ar₁, Ar₂ and R₁ are as defined above; 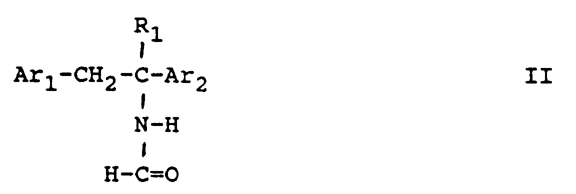
c) for compounds of formula I in which R₂ and R₃ both represent hydrogen, reductive carbamate cleavage of a compound of formula III: 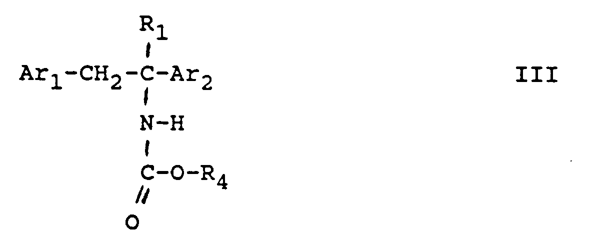 in which Ar₁, Ar₂, and R₁ are as defined above, and R₄ represents an alkyl or aryl group;
d) for compounds of formula I in which R₂ represents COCH₂NH₂, removal of the amine protecting group from a compound of formula IV in which P₁ and P₂ together constitute a suitable protecting group, and Ar₁, Ar₂, R₁ and R₃ are as defined above: 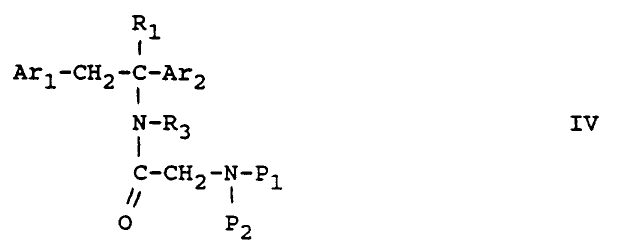
e) for compounds of formula I in which R₂ represents COCH₂NH₂, aminating a compound of formula V in which Ar₁, Ar₂, R₁ and R₃ are as defined above:

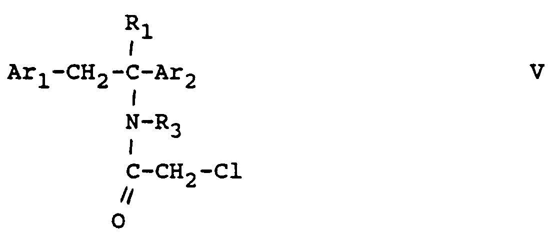

Many alkylation procedures may be used for the reaction of method a), for example a primary amine of formula I in which R₂ and R₃ both represent hydrogen may be reacted with a suitable alkyl halide, for example the alkyl chloride, in an inert solvent such as tetrahydrofuran, in the presence of a base such as triethylamine.

The hydrolysis of method b) may be acid or base catalysed, for example the compound may be treated with 10% hydrochloric acid, and heated at reflux. Compounds of formula II may be prepared by reacting a compound of formula VI: 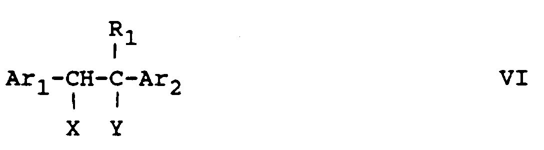
in which Ar₁, Ar₂ and R₁ are as defined above, and either X represents hydrogen and Y represents OH, or X and Y together form a second bond between the carbons to which they are attached, with cyanide ion using conditions under which the nitrogen acts as the nucleophile rather than the carbon. For example, the compound of formula VI may be added to a mixture prepared by adding concentrated sulphuric acid to a suspension of sodium cyanide in glacial acetic acid and n-butylether. The isocyanide thus formed is hydrolysed to the corresponding isonitrile during work-up, upon addition of the reaction mixture to ice. Compounds of formula VI are either commercially available, or may be made from commercially available compounds using known methods.

The reductive cleavage of method c) may be carried out using catalytic hydrogenation conditions, for example using 10%Pd/C as the catalyst and a 1:1 mixture of an alcohol such as methanol and 3N HCl as the solvent at a pressure of about 40psi hydrogen. Alternatively, reducing agents such as zinc dust in a solvent such as 1:9 tetrahydrofuran: acetic acid may be used. Compounds of formula III may be prepared by Curtius rearrangement of a corresponding compound of formula VII: 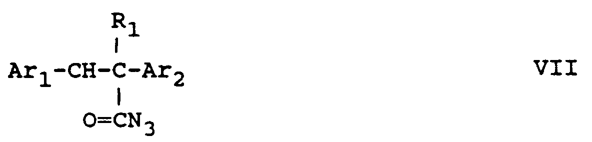
in which Ar₁, Ar₂ and R₁ are as defined above, in the presence of an alcohol of formula R₄OH, where R₄ represents an alkyl or aryl group, for example 2,2,2-trichloroethanol or benzyl alcohol. Compounds of formula VII may be prepared by reacting the corresponding acid with diphenylphosphorylazide in a solvent such as toluene in the presence of a base such as triethylamine. These corresponding acids are either commercially available or may be prepared from commercially available materials using known methods.

For method d), suitable protecting groups that P₁ and P₂ may together constitute include: a urethane protecting group such as benzyloxycarbonyl (CBZ) or t-butyloxycarbonyl (BOC); or P₁ and P₂ together with the nitrogen atom to which they are attached may form a phthalimide group. The amine protecting groups may be removed by either catalytic hydrogenation for the CBZ group, a suitable catalyst being palladium or platinum on carbon, and the reaction being suitably carried out in an inert solvent such as methanol; treatment with an acid such as trifluoroacetic or hydrochloric acid for the BOC group; or treatment with hydrazine in a lower alkanol such as ethanol for the phthalimide group. Compounds of formula IV may be prepared by reacting a compound of formula I in which R₂ represents hydrogen with a compound of formula VIII in which P₁ and P₂ are as defined above: 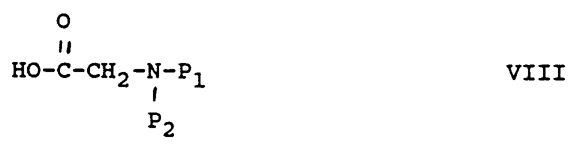
This reaction may be carried out in an inert solvent, such as tetrahydrofuran, in the presence of a coupling reagent such as dicyclohexylcarbodiimide with or without 1-hydroxybenzotriazole or other additives. Compounds of formula VIII are commercially available or may be made by known methods.

For method e), the amination reaction may be carried out by reacting a compound of formula V with ammonia in a solvent such as a lower alkanol, for example methanol or ethanol, or a chlorinated solvent, for example chloroform or methylene chloride, or mixtures thereof. Compounds of formula V may be prepared by reacting a compound of formula I in which R₂ represents hydrogen with an activated two carbon acid derivative which contains a leaving group α to the carbonyl, such as chloroacetyl chloride, in the presence of an acid acceptor, such as triethylamine.

Certain compounds of formula I are novel, thus according to a further aspect of the invention we provide compounds of formula IA: 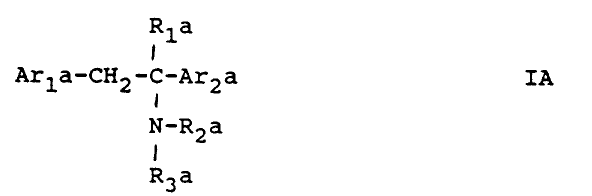
wherein Ar₁a, Ar₂a, R₁a, R₂a and R₃ₐ are defined respectively as Ar₁, Ar₂, R₁, R₂ and R₃ above provided that when R₂a represents hydrogen, then R₁a represents C1 to 6 alkyl;
and pharmaceutically acceptable salts thereof.

A group of compounds of formula IA which may be specifically mentioned is that in which R₁a, R₂a and R₃a are as defined above, and either one or both of Ar₁a and Ar₂a represents 4-hydroxyphenyl.

We prefer that Ar₁a and Ar₂a represent unsubstituted phenyl. When Ar₁a and Ar₂a are substituted, we prefer that they are mono- or disubstituted.

Where R₁a or R₃a or a substituent on an aromatic ring represents alkyl or alkoxy, we prefer that it contains up to 4 carbon atoms, for example ethyl, propyl and especially methyl. R₁a is preferably hydrogen or, more preferably, methyl. R₃a is preferably hydrogen.

Compounds of formula I which are useful in the methods of treatment or prevention of neurological disorders include:
1,2-diphenylethylamine
1,2-diphenyl-2-propylamine
1,2-bis(4-fluorophenyl)-2-propylamine
1,2-diphenyl-2-butylamine
(-)1,2-diphenyl-2-propylamine
(+)1,2-diphenyl-2-propylamine
2,3-diphenyl-2-aminopropanoic acid methyl ester
N-methyl-1,2-diphenyl-2-propylamine
N-methyl-1,2-diphenylethylamine
1-(3-nitrophenyl)-2-phenyl-2-propylamine
1-(3-chlorophenyl)-2-phenyl-2-propylamine
1-(3-bromophenyl)-2-phenyl-2-propylamine
1-(3-cyanophenyl)-2-phenyl-2-propylamine
2-(2-methylphenyl)-1-phenyl-2-propylamine
1-(4-chlorophenyl)-2-phenyl-2-propylamine
1-phenyl-2-(3,4-dichlorophenyl)-2-propylamine
1-phenyl-2-(3-methoxyphenyl)-2-propylamine
1-(4-hydroxyphenyl)-2-phenyl-2-propylamine
1-(4-hydroxyphenyl)-2-phenylethylamine
1-phenyl-2-(4-hydroxyphenyl)ethylamine
1,2-bis(4-hydroxyphenyl)ethylamine
1-phenyl-2-(4-hydroxyphenyl)-2-propylamine
1,2-bis(4-hydroxyphenyl)-2-propylamine
1-(2-pyridinyl)-2-phenylethylamine
1-(3-pyridinyl)-2-phenylethylamine
1-(4-pyridinyl)-2-phenylethylamine
1-phenyl-2-(2-pyridinyl)ethylamine
1-phenyl-2-(3-pyridinyl)ethylamine
1-phenyl-2-(4-pyridinyl)ethylamine
N-methyl-1-(3-pyridinyl)-2-phenylethylamine
3,3,3-trifluoro-1,2-diphenyl-2-propylamine
N-methyl-3,3,3-trifluoro-1,2-diphenyl-2-propylamine
2-amino-N-(1,2-diphenyl-1-methylethyl)acetamide
2-amino-N-(1,2-diphenylethyl)acetamide
2-amino-N-[1,2-bis(4-fluorophenyl)-1-methylethyl]acetamide
The compounds of general formula I are basic compounds and may be used as such or pharmaceutically acceptable acid addition salts may be prepared by treatment with various inorganic or organic acids, such as hydrochloric, hydrobromic, sulfuric, phosphoric, acetic, lactic, succinic, fumaric, malic, maleic, tartaric, citric, benzoic, methanesulfonic or carbonic acids.

For the above mentioned uses the dosage administered will, of course, vary with the compound employed, the mode of administration and the treatment desired. However, in general, satisfactory results are obtained when the compounds are administered at a daily dosage of from about 0.1 mg to about 20 mg per kg of animal body weight, preferably given in divided doses 1 to 4 times a day or in sustained release form. For man the total daily dose is in the range of from 5 mg to 1,400 mg more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, and pharmaceutically acceptable derivatives thereof, may be used on their own or in the form of appropriate medicinal preparations for enteral or parenteral administration.

According to the invention there is also provided a pharmaceutical composition comprising preferably less than 80% and more preferably less than 50% by weight of a compound of formula I, or a pharmaceutically acceptable salt thereof, in admixture with a pharmaceutically acceptable adjuvant, diluent or carrier.

Examples of such adjuvants, diluents and carriers are: for tablets and dragees: lactose, starch, talc, stearic acid; for capsules: tartaric acid or lactose; for injectable solutions: water, alcohols, glycerin, vegetable oils; for suppositories: natural or hardened oils or waxes.

Compositions in a form suitable for oral, i.e. oesophageal administration include tablets, capsules and dragees;
sustained release compositions include those in which the active ingredient is bound to an ion exchange resin which is optionally coated with a diffusion barrier to modify the release properties of the resin.

We prefer the composition to contain up to 50% and more preferably up to 25% by weight of the compound of formula I, or of the pharmaceutically acceptable derivative thereof.

The compounds of formula I and pharmaceutically acceptable derivatives thereof have the advantage that they are less toxic, more efficacious, are longer acting, have a broader range of activity, are more potent, produce fewer side effects, are more easily absorbed or have other useful pharmacological properties, than compounds of similar structure.

The compounds of general formula I possess useful pharmaceutical properties. In particular they possess useful antiepileptic properties, antihypoxia activities and/or NMDA blocking activities. These activities were assessed by standard methods.

Antiepileptic activity was measured by assessing a compound's ability to prevent the hind limb tonic extension component of the seizure in groups of mice induced by maximal electroshock (MES) after oral or intraperitoneal administration according to the procedures of the Epilepsy Branch, NINCDS as published by R. J. Porter, et al, Cleve. Clin. Quarterly 1984, 51, 293, and compared to the standard agents dilantin and phenobarbital. Activities (ED₅₀'s) in the range of 10-400 m/k after oral administration in this assay system were obtained.

The compounds of this invention possess useful antihypoxia activity, that is, they extend the lifetime of animals exposed to a hypoxic environment. This activity is conveniently measured in mice. Groups of mice are tested at various times after the intraperitoneal administration of graded doses of the test compound. The animals' survival time in a temperature controlled hypoxic environment (96% nitrogen and 4% oxygen) is recorded. A statistical comparison is made between coincident vehicle treated animals and the experimental group. The dose-response and minimum active dose (MAD) for compounds are obtained. Other modes of administration can also be used.

NMDA blocking activity was measured by assessing a compound's ability to protect mice from convulsions induced by intravenous administration of 150 m/k of NMDA according to the procedures of Czuczwar et al., (Neurotransmitters, Seizures and Epilepsy III, edited by G. Nistico et al., Raven Press, New York 1986, pages 235-246). Groups of mice were pretreated by 30 min with the test compound by the oral or intraperitoneal routes and then given NMDA. Animals were observed for convulsions as defined by loss of righting reflex. Animals were kept for 60 min after NMDA dosing and mortality was recorded.

NMDA and glycine receptor affinity was also tested in the [³H]L-glutamate and [³H]glycine binding assays following the method of Monaghan & Cotman, PNAS, 83, 7532. (1986) and Watson et al, Neurosci. Res. Comm., 2, 169, (1988).

The following in vitro methods are also used to measure NMDA blocking activity: NMDA (20µM) is applied to tissue slices of rat hippocampus (450µm thick) for approximately 2 minutes causing a massive depolarisation of hippocampal neurons, and a profound reduction of the synaptic field potential. The test is repeated several times to obtain a base line response. The compound under investigation is then included in the buffer bathing the slice, and NMDA is reapplied. The ability of the compound to block the reduction in field potential produced by NMDA is then determined.

In a second in vitro assay, rat hippocampal slices (450µm thick) are pretreated with a buffer containing 10µM 6,7-dinitroquinoxaline-2,3-dione (DNQX) and magnesium (25µM). Under these conditions the synaptic response is almost entirely mediated by NMDA receptors. To evaluate NMDA antagonism, the test compounds are added to the buffer and the synaptic field potentials are compared before and during treatment. The decrease in response caused by the drug is expressed as a percentage of the pre-drug response.

The following compounds of formula I or their acid salts were either commercially available or prepared as described in the aforementioned literature:
1,2-diphenyl-2-propylamine
1,2-bis(4-fluorophenyl)-2-propylamine
1,2-diphenyl-2-butylamine
(-)1,2-diphenyl-2-propylamine
(+)1,2-diphenyl-2-propylamine
2,3-diphenyl-2-aminopropanoic acid methyl ester
N-methyl-1,2-diphenyl-2-propylamine
N-methyl-1,2-diphenylethylamine
1-(2-pyridinyl)-2-phenylethylamine
1-(3-pyridinyl)-2-phenylethylamine
1-(4-pyridinyl)-2-phenylethylamine
1-phenyl-2-(2-pyridinyl)ethylamine
1-phenyl-2-(3-pyridinyl)ethylamine
1-phenyl-2-(4-pyridinyl)ethylamine
N-methyl-1-(3-pyridinyl)-2-phenylethylamine
3,3,3-trifluoro-1,2-diphenyl-2-propylamine
N-methyl-3,3,3-trifluoro-1,2-diphenyl-2-propylamine
Additional examples of the compounds of formula I were prepared as described below.

### Example 1

### Preparation of 1-(4-Hydroxyphenyl)-2-phenyl-2-propylamine hydrochloride

### a) Preparation of 1-[4-(Phenylmethoxy)phenyl]-2-phenyl-2-propylamine maleate

To a stirred suspension of α-methyl-α-[[4-(phenylmethoxy)phenyl]-methyl]benzeneacetic acid (12.9 g, 0.372 mol, prepared by the reaction of the dilithium salt of 2-phenylpropionic acid with 4-(phenylmethoxy)benzyl bromide in benzene (200 mL) under nitrogen were added triethylamine (5.2 mL, 0.037 mol) and diphenylphosphoryl azide (8.4 mL, 0.037 mol) and the solution was heated to reflux for 2 hours. To the reaction was added 2,2,2-trichloroethanol (17.4 mL, 0.18 mol) and the reaction was heated at reflux for 20 h. The reaction mixture was cooled to ambient, diluted with ethyl acetate (100 mL), washed with water (75 mL), lN HCl (75 mL), lN NaOH (75 mL), saturated NaCl (100 mL), dried over magnesium sulfate, and the solvent removed to provide 40.2 g of an oil. This oil was dissolved in tetrahydrofuran (100 mL) and 90% acetic acid (200 mL), and the solution cooled to 5°C. Zinc dust (60 g, 0.92 mol) was added, the mixture was warmed to ambient temperature and stirred overnight. The reaction mixture was filtered and the filtrate was concentrated to an oil. This oil was dissolved in ethyl acetate (200 mL) and water (100 mL), basified with solid sodium carbonate, filtered, the phases separated, and the aqueous phase extracted with ethyl acetate (200 mL). The combined ethyl acetate extracts were washed with saturated NaCl, dried over magnesium sulfate, and the solvent removed to provide 13.1 g of an oil. This oil was purified by silica gel chromatography on a Waters Prep 500, eluting with ammoniated 3%-methanol/methylene chloride to provide 10.6 g of an oil. To a solution of this oil (4.0 g) in ethyl acetate (50 mL) was added maleic acid (1.5 g, 0.013 mol). The solid which formed was isolated by filtration and vacuum dried to provide 2.9 g of 1-[4-(phenylmethoxy)phenyl]-2-phenyl-2-propylamine maleate, mp 180-181°C.

### b) Preparation of 1-(4-Hydroxyphenyl)-2-phenyl-2-propylamine hydrochloride

To a solution of 1-[4-(phenylmethoxy)phenyl]-2-phenyl-2-propylamine (4.2g, 0.013 mol) in methanol (200 mL), lN hydrochloric acid (50 mL) and tetrahydrofuran (50 mL) was added 5% palladium on carbon (0.9 g). The mixture was shaken on a Parr apparatus under a pressure of 35-40 psi of hydrogen for 16 h. The catalyst was removed by filtration and the majority of the solvent removed under vacuum. The residue was basified with lN sodium bicarbonate (100 mL) and extracted with chloroform (4xlOO mL). The combined chloroform extracts were washed with saturated sodium chloride (75 mL) and dried over magnesium sulfate. Removal of solvent gave 3.0 g of an oil. This oil was dissolved in methanol (60 mL), acidified with gaseous HCl and diluted with ether (120 mL). The solid which formed was isolated by filtration and vacuum dried at 70°C for 60 hours to provide 1.6 g of 1-(4-hydroxyphenyl)-2-phenyl-2-propylamine hydrochloride; mp 247-248°C.

### Example 2

### Preparation of 1-(4-Hydroxyphenyl)-2-phenylethylamine hydrochloride

### a) Preparation of 2-Phenyl-1-[4-(phenylmethoxy)phenyl] ethylamine hydrochloride

To a solution of lithium bis(trimethylsilyl)amide (0.047 mol) in tetrahydrofuran (80 mL) and hexane (30 mL) at O°C under nitrogen was added a solution of 4-(phenylmethoxy)benzaldehyde (10.0 g, 0.047 mol). The solution was stirred at approximately 4-10°C for 45 min and a solution of benzylmagnesium chloride (23 mL of 2M THF solution, 0.046 mol) was added. The solution was allowed to warm to ambient temperature and stirred at that temperature overnight. The solution was cooled in an ice-water bath and saturated ammonium chloride (5.7 mL) was added. The precipitate solid was removed by filtration and the filtrate concentrated to give 13.9 g of an oil. This was dissolved in 2-propanol (50 mL) and acidified with gaseous HCl. The white solid which formed was isolated by filtration and dried to give 11.78 g of 2-phenyl-1-[4-(Phenylmethoxy)phenyl]ethylamine hydrochloride; mp 203-204°C.

### b) Preparation of 1-(4-Hydroxyphenyl)-2-phenylethylamine hydrochloride

To a solution of 1-[4-(phenylmethoxy)phenyl]-2-phenylethylamine (4.5 g, 0.013 mol) in methanol (200 mL) and lN hydrochloric acid (50 mL) was added 5% palladium on carbon (0.8 g). The mixture was shaken on a Parr apparatus under a pressure of 35-40 psi of hydrogen for 3 h. The catalyst was removed by filtration, and the filtrate concentrated under vacuum to give a white solid. This solid was recrystallized from 2-propanol (75 mL) and ether (50 mL) and vacuum dried at 80°C for 48 h to give 2.31 g of 1-(4-hydroxyphenyl)-2-phenylethylamine hydrochloride; mp 203-204°C.

### Example 3

### Preparation of 1-Phenyl-2-(4-hydroxyphenyl)ethylamine maleate

### a) Preparation of 1-Phenyl-2-[4-(phenylmethoxy) phenyl]ethylamine hydrochloride

By procedures essentially the same as those described in Example 1a the corresponding 1-phenyl-2-[4-(phenylmethoxy)phenyl]ethylamine hydrochloride 209-210°C, was prepared.

### b) Preparation of 1-Phenyl-2-(4-hydroxyphenyl)ethylamine maleate

To a solution of 1-phenyl-2-[4-(phenylmethoxy) phenyl]ethylamine (4.2 g,0.012 mol) in methanol (150 mL) and lN hydrochloric acid (40 mL) was added 5% palladium on carbon (0.5 g). The mixture was shaken on a Parr apparatus under a pressure of 40 psi of hydrogen for 18 h. The catalyst was removed by filtration, and the filtrate was concentrated under vacuum to give a white solid. Two recrystallizations from 2-propanol, methanol and ether gave 3.68 g of a white solid. The above solid (3.3 g) was partitioned between 1N NaHCO₃ and a mixture of chloroform, methylene chloride and methanol (50-50-15 mL). The aqueous solution was extracted with methylene chloride (2x1OO mL). The combined organic extracts were washed with saturated sodium chloride (75 mL) and dried over magnesium sulfate. Removal of solvent gave 2.3 g of a white solid. The above solid was treated with maleic acid (1.3 g) in ethyl acetate/methanol to provide 2.07 g of 1-phenyl-2-(4-hydroxyphenyl)ethylamine maleate; mp 181-182°C.

### Example 4

### Preparation of 1,2-Bis(4-hydroxyphenyl)ethylamine fumarate

### a) Preparation of 1,2-Bis[(4-phenylmethoxy)phenyl]-2-propylamine hydrochloride

By procedures essentially the same as those described in Example 1a the corresponding 1,2-bis[(4-phenylmethoxy)phenyl]-2-propylamine hydrochloride, mp 185-187°C, was prepared.

### b) Preparation of 1,2-Bis(4-hydroxyphenyl)ethylamine fumarate

To a solution of 1,2-bis[4-(Phenylmethoxy)phenylethyl amine (9.60 g, 0.0216 mol) in methanol (300 mL) and 1N hydrochloric acid (30 mL) was added 5% palladium on carbon (1.1 g). The mixture was shaken on a Parr apparatus under a pressure of 40 psi of hydrogen for 17 h. The catalyst was removed by filtration through celite, and the filtrate was concentrated under vacuum to give 5.6 g of a white solid. The above solid was recrystallized from methanol, isopropanol and ether to give 4.62 g of a white solid.

To a suspension of the above solid (3.6 g) in water (50 mL) were added 1N sodium bicarbonate (50 mL), ethyl acetate (100 mL) and methanol (5 mL). The phases were separated and the aqueous phase was extracted with ethyl acetate (2x1OO mL). The combined organic extracts were washed with saturated sodium chloride (50 mL) and dried over magnesium sulfate. Removal of solvent gave 2.6 g of a white solid. The above solid was dissolved in ethyl acetate (80 mL) and methanol and treated with maleic acid. The solution was diluted with ethyl acetate (30 mL), concentrated to a volume a 40 mL, and again diluted to a volume of 80 mL with ethyl acetate to provide a white solid. This solid was vacuum dried at 60°C for 72 h to provide 2.6 g of 1,2-bis(4-hydroxyphenyl)ethylamine fumarate, mp 275-277°C (D).

### Example 5

### Preparation of 1-Phenyl-2-(4-hydroxyphenyl)-2-propylamine hydrochloride

### a) Preparation of 1-Phenyl-2-[4-(phenylmethoxy)phenyl]-2-propylamine maleate

By procedures essentially the same as those described in Example 1a the corresponding 1-phenyl-2-[4-(phenylmethoxy)phenyl]-2-propylamine maleate, mp 154-155°C, was prepared.

### b) Preparation of 1-Phenyl-2-(4-hydroxyphenyl)-2-propylamine hydrochloride

To a solution of 1-phenyl-2-[4-(phenylmethoxy)phenyl)-2-propylamine (3.50 g, 0.011 mol) in methanol (100 mL), tetrahydrofuran (50 mL) and 1N hydrochloric acid (20 mL) was added 5% palladium on carbon (0.8 g) and the mixture was shaken on a Parr apparatus at approximately 40 psi for 6 h. The catalyst was removed by filtration and the solvent removed under vacuum. Crystallization from 2-propanol and ether and vacuum drying at 80°C for 48 h provided 1.3 g of 1-phenyl-2-(4-hydroxyphenyl)-2-propylamine hydrochloride; mp 160-161°C.

### Example 6

### Preparation of 1,2-Bis(4-hydroxyphenyl)-2-propylamine acetate

### a) Preparation of 1,2-Bis[4-(phenylmethoxy)phenyl]-2-propylamine fumarate

By a procedures essentially the same as those described in Example 1a the corresponding 1,2-bis[4-(phenylmethoxy)phenyl]-2-propylamine fumarate, mp 161-163°C, was prepared.

### b) Preparation of 1,2-Bis(4-hydroxyphenyl)-2-propylamine acetate

To a solution of 1,2-bis[4-(phenylmethoxy)phenyl]-2-propylamine (4.56 g, 0.011 mol) in tetrahydrofuran (200 mL), and acetic acid (50 mL) was added 5% palladium on carbon (0.8 g). The mixture was shaken on a Parr apparatus in an atmosphere of 35-40 psi of hydrogen for 16 h. The catalyst was removed by filtration, and the filtrate concentrated under vacuum to provide an oil (6.6 g). Crystallization of this oil from ethyl acetate and methanol and vacuum drying at 60°C for 48 h provided 1.80 g of 1,2-bis(4-hydroxyphenyl)-2-propylamine acetate; mp 157-159°C.

### Example 7

### Preparation of 1-(3-Nitrophenyl)-2-phenyl-2-propylamine fumarate

To a suspension of sodium cyanide (34.3g, 0.7mol) in glacial acetic acid (500ml) and n-butylether (100ml) at 0°C was added portionwise concentrated sulphuric acid (200ml). The ice bath was removed and a solution of 1-(3-nitrophenyl)-2-phenyl-1-propene (prepared from [(3-nitrophenyl)methylene]triphenylphosphorane and acetophenone, 0.5mol) in n-butylether (100ml) was added dropwise over a period of 2 hours, then the mixture stirred for 48 hours. The mixture was poured into 1000ml ice, and extracted with chloroform. The extracts were washed with water, dried and evaporated to a solid residue which was stirred with hexane (500ml), filtered and dried to give N-formyl-1-(3-nitrophenyl)-2-phenyl-2-propylamine. This was dissolved in methanol (100ml) and 1N HCl (100ml) was added. This mixture was heated to 66-60°C for 24 hours then the solvents were removed and the residue was suspended in 10% NaOH (500ml) and extracted with chloroform (3x300ml). The combined chloroform extracts were dried over magnesium sulphate and the solvent removed to leave an oil. This oil was treated with fumaric acid in ethyl acetate/ isopropanol to give the title compound in 97% yield. Mp 234-235°C.

### Example 8

### Preparation of 1-(3-chlorophenyl)-2-phenyl-2-propylamine maleate

To a solution of N-formyl-1-(3-nitrophenyl)-2-phenyl-2-propylamine (5.0g, 0.017mol) in methanol (200ml) was added 10% Pd/C catalyst (0.5g) and the mixture hydrogenated at 50psi in a Parr apparatus for 3 hours. The catalyst was removed by filtration and the solvent evaporated to a white solid, 4.6g. This solid was recrystallised from isopropanol (50ml) to give 2.6g of N-formyl-1-(3-aminophenyl)-2-phenyl-2-propylamine, mp 114-115°C. To a stirred solution of sodium nitrate (2.0g, 0.03mol) in sulphuric acid (15ml) at 0°C under nitrogen was added dropwise a solution of N-formyl-1-(3-aminophenyl)-2-phenyl-2-propylamine (7.0g, 0.027mol) in acetic acid (75ml) and the solution was stirred 2.5 hours at a temperature less than 20°C. The above mixture was added to a solution of cuprous chloride (5.3g, 0.054mol) in concentrated HCl (50ml) at 15°C, and the resulting solution was stirred at that temperature for 1 hour. The reaction mixture was poured into water (500ml), basified with concentrated ammonium hydroxide, and extracted with chloroform (3x200ml). The combined chloroform extracts were dried over magnesium sulphate and the solvent evaporated to a solid, 7.5g. This solid was recrystallised from isopropanol (20ml) to provide 3.2g of N-formyl-1-(3-chlorophenyl)-2-phenyl-2-propylamine, mp 108-109°C.

To a stirred solution of N-formyl-1-(3-chlorophenyl)-2-phenyl-2-propylamine (5.5g, 0.017mol) in methanol (100ml) was added 1N HCl (100ml) and the mixture was heated to 55-60°C for 24 hours. The solvents were removed, the residue suspended in 10% NaOH (500ml), and extracted with chloroform (3x300ml). The combined chloroform extracts were dried over magnesium sulphate and the solvent removed to provide 4.3g of an oil. This oil was treated with maleic acid in ethyl acetate to provide the title compound, mp 151-152°C.

### Example 9

### Preparation of 1-(3-Bromophenyl)-2-phenyl-2-propylamine maleate

By procedures essentially the same as those described in Example 8, and by substituting cuprous bromide for cuprous chloride and 48% HBr for conc. HCl, the title compound was prepared. Mp 148-149°C

### Example 10

### Preparation of 1-(3-Cyanophenyl)-2-phenyl-2-propylamine maleate

By procedures essentially the same as those described in Example 8, and by substituting cuprous cyanide for cuprous chloride and water for conc. HCl, the title compound was prepared. Mp 157-158°C.

### Example 11

### Preparation of 2-(2-Methylphenyl)-1-phenyl-2-propylamine fumarate

To a stirred solution of o-tolylacetic acid (50.0g, 0.33mol) in tetrahydrofuran (400ml) and hexamethylphosphoric triamide (116ml, 0.668mol) at 0°C was added n-butyllithium (416ml of 1.6M hexane solution, 0.666mol). The solution was warmed to ambient temperature and stirred for 0.5 hour. The solution was cooled to -78°C and iodomethane (20.7ml, 0.33mol) was added and the reaction allowed to warm to room temperature and stirred for 45 minutes. The reaction mixture was cooled to 0°C and n-butyllithium (208ml of 1.6M hexane soltion, 0.333mol) was added. The reaction was warmed to ambient temperature for 10 minutes, recooled to 0°C and benzyl bromide (40.4ml, 0.333mol) was added. The reaction mixture was warmed to ambient temperature and stirred at that temperature overnight. The reaction was poured into 1N HCl and extracted with ethyl acetate. The organic solution was washed with water (2x) saturated sodium chloride, and dried over magnesium sulphate. Removal of the solvent gave 111g of an oil. To a solution of the above oil (111g) in toluene (600ml) were added triethylamine (51ml, 0.36mol) and diphenylphosphoryl azide (82.5ml, 0.38mol) and the solution was heated to reflux under nitrogen for 2 hours. Benzyl alcohol (138ml, 1.3mol) was added and the solution was refluxued overnight. The reaction was cooled to ambient temperature, diluted with ethyl acetate (300ml), washed with 1N HCl (2x), 5% sodium hydroxide (2x), saturated sodium chloride, dried over magnesium sulphate, and the solvent removed to provide 300g of an oil. The above oil (300g) was dissolved in methanol (1.81) and 3N HCl (200ml) and hydrogenated at 40psi of hydrogen over 10% Pd/C (17.4g) for 2 hours. The catalyst was removed by filtration and the solvents evaporated. The residue was dissolved in chloroform (300ml) and washed with 1N sodium carbonate (2x300ml), saturated sodium chloride and dried over magnesium sulphate. Removal of solvent gave an oil which was purified by silica gel chromatography, elution with ammoniated 25% ethyl acetate-hexane, to provide 14.5g of 2-(2-methylphenyl)-1-phenyl-2-propylamine as an oil. The above oil (0.5g) was treated with fumaric acid in ethyl acetate/isopropanol to provide 0.35g of the title compound, mp 180-182°C.

### Example 12

### Preparation of 1-(4-Chlorophenyl)-2-phenyl-2-propylamine maleate

By procedures essentially the same as those described in Example 8, the title compound was prepared, mp 174.5-175.5°C.

### Example 13

### Preparation of 1-Phenyl-2-(3,4-Dichlorophenyl)-2-propylamine maleate

By procedures essentially the same as those described in Example 8, the title compound was prepared, mp 164-165°C.

### Example 14

### Preparation of 1-Phenyl-2-(3-methoxyphenyl)-2-propylamine maleate

By procedures essentially the same as those described in Example 11, the title compound was prepared, mp 139-140°C.

### Example 15

### Preparation of 1-Phenyl-1-[3-((2,2,2-trichloroethoxycarbonyl)amino)phenyl]-2-propylamine tosylate

By procedures essentially the same as those described in Example 7, the title compound was prepared. Mp 237-239°C.

### Example 16

### Preparation of 2-(3-Chlorophenyl)-1-phenyl-2-propylamine maleate

By procedures essentially the same as those described in Example 8, the title compound was prepared. Mp 174-175°C.

### Example 17

### Preparation of 1-(2-Chlorophenyl)-2-phenyl-2-propylamine maleate

By procedures essentially the same as those described in Example 8, the title compound was prepared. Mp 169-171°C.

### Example 18

### Preparation of 2-(2-Chlorophenyl)-1-phenyl-2-propylamine fumarate

By procedures essentially the same as those described in Example 8, the title compound was prepared. Mp 191-193°C.

### Example 19

### Preparation of 2-(3-Nitrophenyl)-1-phenyl-2-propylamine maleate

By procedures essentially the same as those described in Example 8, the title compound was prepared. Mp 152-153°C.

### Example 20

### Preparation of 2-(4-Chlorophenyl)-1-phenyl-2-propylamine maleate

By procedures essentially the same as those described in Example 8, the title compound was prepared. Mp 171.5-173°C.

### Example 21

### Preparation of 2-(3,4-Dimethoxyphenyl)-1-phenyl-2-propylamine fumarate

By procedures essentially the same as those described in Example 8, the title compound was prepared. Mp 185°C (D).

### Example 22

### Preparation of 2-(4-methylphenyl)-1-phenyl-2-propylamine maleate

By procedures essentially the same as those described in Example 8, the title compound was prepared. Mp 169°C (D).

### Example 23

### Preparation of 2-(4-methoxyphenyl)-1-phenyl-2-propylamine maleate

By procedures essentially the same as those described in Example 8, the title compound was prepared. Mp 169-170°C.

### Example 24

### Preparation of 1-(3,4-Dichlorophenyl)-1-phenyl-2-propylamine maleate

By procedures essentially the same as those described in Example 8, the title compound was prepared. Mp 175-176°C.

### Example 25

### Preparation of 1-(4-methoxyphenyl)-2-phenyl-2-propylamine maleate

By procedures essentially the same as those described in Example 8, the title compound was prepared. Mp 167-168°C.

### Example 26

### Preparation of 2-Amino-N-[1-(3-chlorophenyl)-2-phenyl-1-methylethyl]acetamide maleate

To a stirred solution of 2-(3-chlorophenyl)-1-phenyl-2-propylamine (9.0g, 0.037mol) in chloroform (100ml) under nitrogen was added N-(tert-butoxycarbonyl)glycine (7.0g, 0.04mol), and then a solution of dicyclohexylcarbodiimide (8.2g, 0.04mol) in chloroform (50ml) and the mixture stirred for 20 hours. The precipitated solid was removed by filtration and the solvent evaporated. The residue was dissolved in ethyl acetate (200ml) and filtered. The filtrate was washed with 1N HCl (100ml), 2N sodium carbonate (100ml) and saturated NaCl (100ml), dried, then evaporated to an oil, 14.2g. The above oil was dissolved in ethyl acetate (250ml), cooled in an ice water bath and the solution was acidified with gaseous HCl. The solution was warmed to ambient temperature and stirred overnight. The solvent was evaporated and the residue was partitioned between chloroform (200ml) and 3% NaOH (100ml). The chloroform solution was washed with saturated NaCl (100ml) and dried over magnesium sulphate. Removal of the solvent gave 10.7g of an oil. Treatment of this oil with maleic acid (4.1g, 0.035mol) in ethyl acetate (300mnl) gave a white solid which was vacuum dried at 60°C for 60 hours to give 10.7g of the title compound, mp 160 - 161°C.

### Example 27

### Preparation of 2-Amino-N-[2-(2-chlorophenyl)-1-phenyl-1-methylethyl]acetamide maleate

By procedures essentially the same as those described in Example 26 the title compound was prepared. Mp 119 - 123°C.

### Example 28

### Preparation of 2-Amino-N-[2-(4-chlorophenyl)-1-phenyl-1-methylethyl]acetamide maleate

By procedures essentially the same as those described in Example 26 the title compound was prepared. Mp 162 - 164°C.

### Example 29

### Preparation of 2-Amino-N-[1-(2-methylphenyl)-2-phenyl-1-methylethyl]acetamide fumarate

By procedures essentially the same as those described in Example 26 the title compound was prepared. Mp 203°C (D).

### Example 30

### Preparation of 2-Amino-N-[1-(2-chlorophenyl)-2-phenyl-1-methylethyl]acetamide fumarate

By procedures essentially the same as those described in Example 26 the title compound was prepared. Mp 209°C (D).

### Example 31

### Preparation of 2-Amino-N-[2-(3-chlorophenyl)-1-phenyl-1-methylethyl]acetamide maleate

By procedures essentially the same as those described in Example 26 the title compound was prepared. Mp 171 - 172°C.

### Example 32

### Preparation of 2-Amino-N-[2-(3-aminophenyl)-1-phenyl-1-methylethyl]acetamide fumarate

By procedures essentially the same as those described in Example 26 the title compound was prepared. Mp 189 - 190°C.

### Example 33

### Preparation of 2-Amino-N-[2-(3-bromophenyl)-1-phenyl-1-methylethyl]acetamide maleate

By procedures essentially the same as those described in Example 26 the title compound was prepared. Mp 168 - 169°C.

### Example 34

### Preparation of 2-Amino-N-[2-(3-nitrophenyl)-1-phenyl-1-methylethyl]acetamide maleate

To a stirred solution of 1-(3-nitrophenyl)-2-phenyl-2-propylamine (10.0g, 0.039mol) in dichloromethane (250ml) under nitrogen was added N-phthaloylglycine (8.87g, 0.043mol), and then a solution of dicyclohexylcarbodiimide (8.4g, 0.041mol) in dichloromethane (75ml) and the mixture stirred for 20 hours. The precipitated solid was removed by filtration and the solid evaporated to give 22.3g of a white solid. This solid was slurried in hot ethanol (200ml) to provide 16.4g of 2-(1,3-dioxoisoindol-2-yl)-N-[2-(3-nitrophenyl)-1-phenyl-1-methylethyl]acetamide, mp 172 - 173°C. To a stirred suspension of the above compound (8.0g, 0.018mol) in absolute ethanol (150ml) was added 65% hydrazine hydrate (2.0ml, 0.054mol) and the mixture was heated to 40°C for 48 hours. The precipitated solid was removed by filtration and the solvent evaporated. The residue was dissolved in 3% NaOH (200ml) and extracted with chloroform (3 x 100ml). The combined chloroform extracts were dried over magnesium sulphate and the solvent evaporated to an oil 4.3g. The above oil was dissolved in ethyl acetate (200ml) and treated with maleic acid (1.7g, 0.015mol). The white solid which formed was isolated by filtration and vacuum dried at 50°C for 128 hours to provide 3.7g of the title compound, mp 170 - 171°C.

### Example 35

### Preparation of 2-Amino-N-[2-phenyl-1-(3-nitrophenyl)-1-methylethyl]acetamide maleate

By procedures essentially the same as those described in Example 34, the title compound was prepared. Mp 165 - 167°C.

### Example 36

### Preparation of 2-Amino-N-[1-(3-aminophenyl)-2-phenyl-1-methylethyl]acetamide maleate

To a stirred solution of 1-phenyl-2-[3-[(2,2,2-trichloroethoxycarbonyl)amino]phenyl]-2-propylamine (9.9g, 0.025mol) in chloroform (100ml) under nitrogen were added N-CBZ-glycine (5.7g, 0.027mol) and then a solution of dicyclohexylcarbodiimide (5.6g, 0.027mol) in chloroform (70ml) and the mixture was stirred for 18 hours. The precipitated solid was removed by filtration and the solvent evaporated. The residue was dissolved in ethyl acetate (200ml) and filtered. The filtrate was washed with 1N HCl (100ml), 2N sodium carbonate (100ml), saturated NaCl (100ml), dried and evaporated to an oil, 15.3g.

To a solution of the above oil in tetrahydrofuran (150ml) and 90% acetic acid (150ml) was added zinc dust (25g, 0.38mol) and the mixture stirred at ambient temperature for 20 hours. The reaction mixture was filtered, the filtrate dissolved in water (400ml) and ethyl acetate (300ml) and this mixture basified with solid sodium carbonate. The phases were separated and the aqueous phase was extracted with ethyl acetate (200ml). The combined organic extracts were washed with saturated NaCl (200ml) and dried over magnesium sulphate. Removal of solvents gave 10.3g of an oil. This oil was purified by silica gel chromatography on a Waters prep. HPLC, eluting with 50% ethyl acetate/hexane to provide 7.3g of an oil. This oil was dissolved in methanol (225ml) and 1N HCl (75ml), and hydrogenated at 40psi in a Parr apparatus over 1.8g of 5% Pd/C catalyst for 2 hours. The catalyst was removed by filtration, and the solvent was evaporated. The residual oil was dissolved in 1N sodium carbonate (150ml) and extracted with chloroform (3 x 100ml). The combined extracts were washed with satureted NaCl (100ml), dried over magnesium sulphate, and the solvent evaporated to provide an oil, 4.5g. This oil was dissolved in ethyl acetate (75ml) and treated with maleic acid (2.1g, 0.018mol). The white solid which formed was isolated by filtration and vacuum dried at 60°C for 48 hours to provide 5.15g of the title compound, mp 148 - 150°C.

### Example 37

### Preparation of 2-Amino-N-[1-(4-chlorophenyl)-2-phenyl-1-methylethyl]acetamide maleate

By procedures essentially the same as those described in Example 26, the title compound was prepared. Mp 172.5 - 175.5°C.

### Example 38

### Preparation of 2-Amino-N-[1-(3,4-dichlorophenyl)-2-phenyl -1-methylethyl]acetamide maleate

By procedures essentially the same as those described in Example 26, the title compound was prepared. Mp 186.5 - 187.5°C.

### Example 39

### Preparation of 2-Amino-N-[1-(3,4-dimethoxyphenyl)-2-phenyl -1-methylethyl]acetamide maleate

By procedures essentially the same as those described in Example 26, the title compound was prepared. Mp 174.5°C (D).

### Example 40

### Preparation of 2-Amino-N-[2-(3-cyanophenyl)-1-phenyl -1-methylethyl]acetamide maleate

By procedures essentially the same as those described in Example 26, the title compound was prepared. Mp 165 - 166°C.

### Example 41

### Preparation of 2-Amino-N-[1-(4-methylphenyl)-2-phenyl -1-methylethyl]acetamide maleate

By procedures essentially the same as those described in Example 26, the title compound was prepared. Mp 163°C.

### Example 42

### Preparation of 2-Amino-N-[2-phenyl-1-(4-hydroxyphenyl)-1-methylethyl]acetamide maleate

To a stirred solution of 1-phenyl-2-[4-(phenylmethoxy)phenyl]-2-propylamine (3.77g, 0.012mol) in dichloromethane (50ml) under nitrogen were added N-CBZ-glycine (2.47g, 0.012mol) and then a solution of dicyclohexylcarbodiimide (2.45g, 0.012mol) in dichloromethane (25ml), and the mixture stirred for 14 hours. The precipitated solid was removed by filtration and the solvent evaporated. The residue was dissolved in ethyl acetate (100ml) and filtered again. The ethyl acetate solution was washed with 1N HCl, 1N sodium hydroxide, saturated NaCl, dried over magnesium sulphate and the solvent evaporated to give 6.1g of an oil. This oil was dissolved in ethanol (150ml), tetrahydrofuran (25ml) and 1N HCl (50ml), and hydrogenated at 40psi in a Parr apparatus over 5% Pd/C (0.8g) for 4 hours. The catalyst was removed by filtration and the solvents evaporated to give a solid. This solid was suspended in 1N sodium carbonate (100ml) and extracted with ethyl acetate (250ml) containing methanol (50ml). The organic solution was washed with saturated sodium chloride solution, dried over magnesium sulphate and the solvent evaporated to leave a white solid, 1.82g. This solid was dissolved in methanol (100ml) and maleic acid (0.74g, 0.0064mol) was added. The solvent was evaporated to an oil. This oil was crystallised from ethyl acetate-methanol and vacuum dried to provide 1.91g of the title compound, mp 174 - 175°C.

### Example 43

### Preparation of 2-Amino-N-[1-(4-hydroxyphenyl)-2-phenylethyl]acetamide maleate

By procedures essentially the same as those described in Example 42 the title compound was prepared. Mp 184-185°C.

### Example 44

### Preparation of 2-Amino-N-[2-(4-hydroxyphenyl)-1-phenyl-1-methylethyl]acetamide maleate

By procedures essentially the same as those described in Example 42 the title compound was prepared. Mp 167 - 168°C.

### Example 45

### Preparation of 2-Amino-N-[2-(4-hydroxyphenyl)-1-phenylethyl]acetamide acetate

To a stirred solution of 2-[4-(phenylmethoxy)phenyl)-1-phenylethylamine (6.51g, 0.022mol) in chloroform (100ml) under nitrogen were added N-CBZ-glycine (4.35g, 0.021mol) and then a solution of dicyclohexylcarbodiimide (4.42g, 0.021mol) in chloroform (50ml) and the mixture stirred for 24 hours. The precipitated solid was removed by filtration. The filter cake was suspended in warm tetrahydrofuran (150ml) and the insoluble material was removed by filtration. The filtrates were combined and the solvents evaporated to a white solid, 10.7g. This solid was recrystallised from ethyl acetate and cyclohexane to provide 8.5g of a white solid, mp 155 - 157°C. This solid was dissolved in tetrahydrofuran (300ml) and acetic acid (70ml) and hydrogenated at 40psi in a Parr apparatus over 5% Pd/C (1.5g) for 17 hours. The catalyst was removed by filtration and the solvent evaporated to leave a white solid. This solid was recrystallised twice from ethyl acetate amd methanol, and vacuum dried to provide 2.53g of the title compound, mp 145 - 147°C.

### Example 46

### Preparation of 2-Amino-N-[1,2-bis(4-hydroxyphenyl)-1-methylethyl]acetamide acetate

By procedures essentially the same as those described in Example 45 the title compound was prepared. Mp 225 - 228°C (D).

### Example 47

### Preparation of 2-Amino-N-[1,2-bis(4-hydroxyphenyl)ethyl] acetamide acetate

By procedures essentially the same as those described in Example 45 the title compound was prepared. Mp 192 - 193°C.

### Example 48

### Preparation of 2-Amino-N-[1-(3-methoxyphenyl)-2-phenyl-1-methylethyl]acetamide maleate

By procedures essentially the same as those described in Example 45 the title compound was prepared. Mp 150°C.

### Example 49

### Preparation of 2-Amino-N-[1-(4-methoxyphenyl)-2-phenyl-1-methylethyl]acetamide maleate

By procedures essentially the same as those described in Example 34 the title compound was prepared.Mp 157-158°C.

### Example 50

### Preparation of 2-Amino-N-[2-(3,4-dichlorophenyl)-1-phenyl-1-methylethyl]acetamide maleate

By procedures essentially the same as those described in Example 26 the title compound was prepared. Mp 172.5 - 174°C.

### Example 51

### Preparation of 2-Amino-N-[2-(4-methoxyphenyl)-1-phenyl-1-methylethyl]acetamide maleate

By procedures essentially the same as those described in Example 34 the title compound was prepared. Mp 165 - 167°C.

## Claims

1. (*S*)-1-phenyl-2-(2-pyridinyl)ethylamine, or a pharmaceutically acceptable salt thereof.

2. A compound as claimed in claim 1, which is (*S*)-1-phenyl-2-(2-pyridinyl)ethylamine dihydrochloride.

3. A compound as claimed in claim 1, which is (*S*)-1-phenyl-2-(2-pyridinyl)ethylamine benzoate.

4. A compound as claimed in claim 1, which is (*S*)-1-phenyl-2-(2-pyridinyl)ethylamine malate.

5. (*S*)-1-phenyl-2-(2-pyridinyl)ethylamine, or a pharmaceutically acceptable salt thereof, for use as a pharmaceutical.

6. A pharmaceutical formulation including (*S*)-1-phenyl-2-(2-pyridinyl)ethylamine, or a pharmaceutically acceptable salt thereof, in admixture with a pharmaceutically acceptable adjuvant, diluent or carrier.

7. The use of (*S*)-1-phenyl-2-(2-pyridinyl)ethylamine, or a pharmaceutically acceptable salt thereof, as active ingredient in the manufacture of an anticonvulsant or neuroprotective medicament.

8. A process for the production of (*S*)-1-phenyl-2-(2-pyridinyl)ethylamine, or a pharmaceutically acceptable salt thereof, which comprises resolution of 1-phenyl-2-(2-pyridinyl)ethylamine.
